# EUROPÄISCHE PATENTSCHRIFT

(11) **EP 2 785 761 B1**
(45) Veröffentlichungstag und Bekanntmachung des Hinweises auf die Patenterteilung: **13.09.2017**
(21) Anmeldenummer: 12791509.8
(22) Anmeldetag: 29.11.2012
(51) Int. Cl.: C08G 18/78, C07C 273/18, C08G 18/79, C08G 18/09

(54) **VERFAHREN ZUR KONTINUIERLICHEN ISOCYANATMODIFIZIERUNG**
PROCESS FOR CONTINUOUS ISOCYANATE MODIFICATION
PROCÉDÉ POUR MODIFIER EN CONTINU DES ISOCYANATES

(30) Priorität: 30.11.2011 EP 11191377
(43) Veröffentlichungstag der Anmeldung: 08.10.2014
(73) Patentinhaber: Covestro Deutschland AG, 51373 Leverkusen (DE)
(72) Erfinder: RICHTER, Frank, 51373 Leverkusen (DE); ANSTOCK, Martin, 47800 Krefeld (DE); BAHKE, Philip, 77019 Houston, TX (US); GOSCH, Andreas, 44789 Bochum (DE); HALPAAP, Reinhard, 51519 Odenthal (DE); HECKING, Andreas, 40764 Langenfeld (DE); BUCHHOLZ, Sigurd, 50767 Köln (DE); TRACHT, Ursula, 51379 Leverkusen (DE); DOBRE, Adrian Cosmin, 50735 Köln (DE)
(74) Vertreter: Levpat
(86) Internationale Anmeldenummer: PCT/EP2012/073998
(87) Internationale Veröffentlichungsnummer: WO 2013/079614

(56) Entgegenhaltungen:
- EP-A1- 0 017 998
- DE-A1-102004 060 131
- US-A1- 2011 218 314

## Beschreibung

Die Oligo- bzw. Polymerisierung von Isocyanaten, insbesondere Diisocyanaten, hier zusammenfassend Modifizierung genannt, ist seit langem bekannt. Enthalten die modifizierten Polyisocyanate freie NCO-Gruppen, die ggf. auch mit Blockierungsmitteln vorübergehend desaktiviert worden sein können, sind sie außerordentlich hochwertige Ausgangsstoffe für die Herstellung einer Vielzahl von Polyurethan-Kunststoffen und Beschichtungsmitteln.

Es haben sich eine Reihe technischer Verfahren zur Isocyanatmodifizierung etabliert, wobei man in der Regel das zu modifizierende Isocyanat, meist ein Diisocyanat, durch Zusatz von Katalysatoren umsetzt und diese anschließend, wenn der gewünschte Umsatzgrad des zu modifizierenden Isocyanates erreicht ist, durch geeignete Maßnahmen unwirksam macht (deaktiviert oder abtrennt) und anschließend oder simultan das erhaltene Polyisocyanat in der Regel vom nicht umgesetzten Monomer abtrennt. Letzteres geschieht meist destillativ. Das destillativ zurückgewonnene Monomer (in der Regel das Ausgangs-Diisocyanat) wird anschließend wieder in der gleichen Weise umgesetzt. Eine Zusammenstellung dieser Verfahren des Standes der Technik findet sich in H. J. Laas et al., J. Prakt. Chem. 1994, 336, 185 ff.

Spezielle Formen der Isocyanatmodifizierung sind die Dimerisierung und Trimerisierung, wobei man nur ein Isocyanat-Dimer (Uretdion) aber zwei Gruppen von Isocyanat-Trimeren kennt: Isocyanurate und Iminooxadiazindione (zuweilen auch unsymmetrische Trimere oder asymmetrische Trimere genannt). Iminooxadiazindione weisen gegenüber den isomeren Isocyanuraten (auf Basis des gleichen Ausgangsmaterials) bei ansonsten weitgehend identischen Eigenschaften wie Molekulargewicht(sverteilung) und NCO-Gehalt eine signifikant niedrigere Viskosität auf und eignen sich daher besonders zur Herstellung von lösemittel-armen- und -freien Polyisocyanaten, insbesondere für den Lackrohstoff- und Beschichtungsmittelsektor. Auch wenn die durch Diisocyanat-"Trimerisierung" entstehenden Polyisocyanate neben den nur einen Isocyanurat- bzw. Iminooxadiazindionring ("Idealstruktur, n=3, n steht im Text für die Anzahl der im jeweiligen Oligomermolekül eingebauten monomeren Diisocyanateinheiten) aufweisenden Molekülen höhermolekulare Komponenten (n=5, 7, 9 u.s.w.) enthalten, in denen ggf. auch beide Strukturtypen (Isocyanurate und Iminooxadiazindione) eingebaut sein können, wird hier zur Bezeichnung dieser Oligomerenmischungen allgemein nur von "Isocyanattrimeren" gesprochen. Entsprechendes gilt für die Uretdionbildung ("Dimerisierung"). Man erhält in der Regel bei der Trimerisierung immer geringe Anteile an Uretdiongruppen tragenden Oligomerbestandteilen und umgekehrt ist die Isocyanat-Dimerisierung nur schwer so zu führen, dass keine Isocyanat-Trimeren entstehen. Die Bezeichnung der Verfahrensprodukte "Trimer" (optional mit dem Präfix "asymmetrisch") vs. "Dimer" richtet sich mithin immer nach der Art der ganz überwiegend im Oligomerengemisch vorliegenden Strukturtypen, was sich leicht mittels IR- und/oder NMR-Spektroskopie oder anderer analytischer Verfahren ermitteln läßt.

Als Katalysatoren für die Modifizierung von Isocyanaten haben sich eine Reihe von Verbindungen bewährt, vgl. H. J. Laas et al., J. Prakt. Chem. 1994, 336, 185 ff, EP 2100886, EP 2067773, EP 1982979; EP 1645577; EP 1533301, EP 1422223, EP 962455, EP 962454, EP 896009, EP 798299, EP 447074, EP 379914, EP 339396, EP 315692, EP 295926 sowie EP 235388.

Besonders geeignet für die Dimerisierung sind kovalent aufgebaute Substanzen mit dreiwertigem Phosphor als Zentralatom, der nicht Teil eines Cyclus ist, sowie bestimmte Aminopyridine.

Besonders geeignet für die Trimerisierung sind spezielle Phosphacyclen mit dreiwertigem P-Atom und salzartig aufgebaute Substanzen mit Kationen, die für eine gute Löslichkeit im Isocyanatmedium sorgen, besonders Tetraorganyl-ammonium und -phosphonium, und Anionen, die sich durch H⁺-Abstraktion aus schwachen Säuren wie z.B. Carbonsäuren, Phenolen, Alkoholen, Wasser, HF etc. ableiten. Fluoride, Hydrogendifluoride, Dihydrogentrifluoride und höhere Polyfluoride, wie auch die Anionen spezieller, fluorierte Alkylgruppen tragender Carbonsäuren, führen verstärkt zur Bildung hoch Iminooxadiazindiongruppen-haltiger Isocyanat-Trimerer.

Ein Nachteil dieser Verfahren des Standes der Technik ist, dass sich die als Katalysator verwendeten Spezies häufig unter Bildung störender Nebenprodukte zersetzen, was sich bei den Ammoniumsalzen durch die Gegenwart tertiärer Amine in den Verfahrensprodukten negativ bemerkbar macht, der sich z.B. in einem unangenehmen Amingeruch der Polyisocyanate und Problemen bzgl. der Langzeitstabilität derartiger Produkte äußern kann und bei den Phosphoniumsalzen durch einen sukzessiv ansteigenden Phosphorgehalt des - in der Regel destillativ - wiedergewonnenen Monomers (Recyclat) äußert, was nachteilig für die Prozeßstabilität ist.

Zwar lassen sich so verunreinigte Destillatströme aufreinigen, vgl. EP 1939171, jedoch ist eine derartige Vorgehensweise mit zusätzlichem Aufwand verbunden, den es zu vermeiden gilt.

Verstärkt treten diese negativen Begleiterscheinungen bei der kontinuierlichen Isocyanatmodifizierung z.B. in einer Mehrkesselkaskade auf, da hier - bedingt durch kurze Verweilzeiten und/oder höhere Reaktionstemperaturen - in der Regel mit höheren Katalysatormengen (bezogen auf das zu modifizierende Isocyanat) gearbeitet werden muss, als bei diskontinuierlicher ("batch-") Fahrweise.

Hinzu kommt, dass bei kontinuierlicher Reaktionsführung häufig eine breitere Molekulargewichtsverteilung der Verfahrensprodukte in Kauf genommen werden muss, die sich in einer höheren Viskosität und einem niedrigeren Isocyanatgehalt der Polyisocyanate niederschlägt, was wiederum Umsatzeinbußen gegenüber dem diskontinuierlichen ("batch"-) Verfahren nach sich zieht, so man die Herstellung von Polyisocyanaten mit gleichen Produkteigenschaften (insbes. Viskosität und NCO-Gehalt) anstrebt.

Verwendet man stark verdünnte Katalysatorlösungen in gegenüber Isocyanaten reaktiven Katalysatorlösungsmitteln, typischerweise mono-, di- oder - weniger üblich - trifunktionellen Alkoholen, kommt ein weiteres Problem hinzu: die bestimmungsgemäße (Haupt-)Reaktion, die Isocyanatmodifizierung, kann zwar durch frühzeitigeren Abbruch der Reaktion bei niedrigeren Umsatzstufen terminiert werden, die Reaktion des Katalysatorlösungsmittels mit den freien Isocyanatgruppen aller im Reaktionsgemisch vorliegender Spezies hingegen nicht, sie läuft immer mindestens bis zur Stufe des Urethans (Carbamates) ab, wobei aber in der Regel erwünscht ist, den Umsatz des Alkohols bis zur Stufe des Allophanates zu führen, was die Gegenwart des aktiven Trimerisierungskatalysators, der in der Regel auch für die Allophanatbildung geeignet und erforderlich ist, bedingt. Allophanate sind gegenüber den aus gleichen Bausteinen aufgebauten Urethanen besser im Polyisocyanat löslich als die entsprechenden Urethane und weisen - zumindest im Fall der Produkte auf Basis monofunktioneller Alkohole - eine gegenüber den Trimeren deutlich niedrigere Viskosität auf Prinzipiell nachteilig ist hingegen, dass die aus monofunktionellen Alkoholen und Diisocyanaten aufgebauten Allophanate nur NCO-difunktionell sind und somit die (mittlere) NCO-Funktionalität der Polyisocyanate erniedrigen.

Kontinuierliche Prozessführungen sind in der Polymerchemie nicht neu, vgl. EP 1861428 und darin zitierter Stand der Technik. Der Lehre der EP 1861428 ist jedoch kein Hinweis auf die Durchführung des speziellen Verfahrens der Isocyanat-Oligomerisierung zu entnehmen. So wird in EP 1861428 ganz allgemein vom Mischen mindestens zweier Lösungen gesprochen, die Reaktanden enthalten. Sieht man von der vorstehend geschilderten, im untergeordneten Maße ablaufenden Reaktion des Katalysatorlösungsmittels, so es denn überhaupt NCOreaktive funktionelle Gruppen enthält, ab, ist die Isocyanatmodifizierung durch die Reaktion lediglich eines Reaktanden gekennzeichnet. Weiterhin wird nach der Lehre der EP 1861428 in Gegenwart von Ultraschall gearbeitet, was bei der technischen Umsetzung des Verfahrens mit zunehmender Anlagengröße auf signifikante Schwierigkeiten stößt.

Eine kontinuierliche Verfahrensführung wird ganz allgemein in vielen Patentschriften zur (Di)Isocyanat-Modifizierung als geeignete Ausführungsform des Verfahrens angegeben, jedoch ohne konkrete Hinweise auf die Lösung der o.g. Probleme zu liefern.

Insbesondere beschreibt die DE 10232573 ein Verfahren zur kontinuierlichen Herstellung Isocyanuratgruppen enthaltender Polyisocyanate mit aromatisch gebundenen Isocyanatgruppen, wobei die Reaktion in Rohrreaktoren mit turbulenter Strömung bei Reynoldszahlen oberhalb 2300 oder in Reaktoren mit Mischorganen bei einem Leistungseintrag oberhalb 0,2 W/1 ablaufen soll. Diese Herangehensweise hat folgende Nachteile:
- Die geforderte hohe Reynoldszahl bedingt einen hohen Durchsatz im Rohrreaktor und verlangt bei ansteigender Viskosität, die sich mit steigendem Umsatz bei der (Di)Isocyanattrimerisierung zwangsläufig einstellt, in der Regel nach einer Erhöhung der Reaktionstemperatur um das Kriterium der Turbulenz zu erfüllen. Somit ist die Reaktion weder in Richtung geringerer Produktdurchsätze noch zu niedrigeren und vor allem über die gesamte Reaktionsstrecke konstanten Reaktionstemperaturen beliebig skalierbar und würde dann - bei Unterschreitung der für Turbulenz nötigen Reynoldszahl (sei es durchsatz- oder temperaturbedingt) - zu Produkten mit gegenüber dem batch-Ansatz deutlich verbreiterter Molekulargewichtsverteilung führen.
- Umsetzungen in Reaktoren mit Mischorganen werden in DE 10232573 [0016] insbesondere am Beispiel von gerührten Zellreaktoren beschrieben. Diese weisen immer eine - insbesondere bei geringem Leistungseintrag über das Mischorgan - nicht zu vernachlässigende Rückvermischung auf, weshalb in DE 10232573 [0014] explizit auf den Leistungseintrag (oberhalb 0,2 W/1, bevorzugt oberhalb 0,3 W/1, besonders bevorzugt 0,5 W/1 und ganz besonders bevorzugt 0,8 W/1) eingegangen wird. Eine derartige Abhängigkeit des Ergebnisses der Modifizierungsreaktion vom Leistungseintrag ist generell nachteilig.
- Die Umsetzung aliphatischer Isocyanate wird nicht beschrieben.
- Eine Zurückführung nicht umgesetzten Monomers nach Teilumsatz der Monomeren wird nicht beschrieben, mithin konnten aus der Lehre der DE 10232573 keinerlei Aussagen zum Verhalten der Katalysator-folge und -zersetzungsprodukte in einem Verfahren mit nur anteiligem Monomerumsatz und anschließender Rückgewinnung desselben abgeleitet werden.

Der EP 17998 ist u.a. der Hinweis auf die Durchführung der Trimerisierung in einem "kühlbaren, rohrförmigen Reaktor unter gleichzeitiger Zudosierung von katalysatorhaltigem monomerem Isocyanat" (EP 17998, Anspruch 3) zu entnehmen. Die Dosierung eines vorgefertigten Gemisches aus Katalysator und zu trimerisierendem Isocyanat ist jedoch generell von Nachteil und gelingt nur, wenn man entweder wenig reaktive Isocyanate, z.B. Cycloaliphaten oder sterisch gehinderte Linearaliphaten einsetzt, auf die die EP 17998 im überwiegenden Teil der Durchführungsbeispiele abhebt, oder wenn der Katalysator nach Zugabe zum Isocyanat nicht unmittelbar die - stark exotherme - Trimerisierung bewirkt, sondern eine gewisse Latenzzeit aufweist. Beides schränkt das in EP 17998 beschriebene Verfahren stark ein. Hinzu kommt, wie man den Beispielen der EP 17998 entnehmen kann, dass das Isocyanat-Katalysator-Gemisch bereits vor dem Eintritt in die Reaktionsstrecke zu 7,5 - 10 % trimerisiert ist. Niedrige Umsätze, die gerade heutzutage häufig bevorzugt sind, da sie zu niedrigviskosen Produkten führen, die vor dem Hintergrund immer stringenter werdender Anforderungen an den Emmisionsschutz (Stichwort VOC) an Bedeutung gewinnen, sind mithin nach der beschriebenen Methode des Standes der Technik nicht realisierbar.

Hinzu kommt, wie man den Beispielen der EP 17998 entnehmen kann, die sich auf die kontinuierliche Durchführung der Trimerisierung von Hexamethylendiisocyanat (HDI) beziehen (EP 17998 Bsp. B4, B5 und B6, s. auch Tab. IIa dort), dass die Molekulargewichtsverteilung der erhaltenen Produkte deutlich breiter sein muss, als sie im batch-Experiment bei vergleichbarem Umsatz, gekennzeichnet durch den Brechungsindex der Rohware vor destillativer Abtrennung des Monomers, gewesen wäre: keines der in EP 17998 angeführten Beispiele zu HDI-Trimeren liefert Produkte mit einem NCO-Gehalt oberhalb 21,4 % - s. hierzu auch die Vergleichsbeispiele der vorliegenden Schrift.

Schließlich beansprucht die DE 10 2004 06 0131 ein kontinuierliches Verfahren zur partiellen Trimerisierung von (cyclo)aliphatischen Isocyanaten in Gegenwart mindestens eines Katalysators, dadurch gekennzeichnet, dass man das Verfahren zumindest teilweise in mindestens zwei rückvermischten Reaktionszonen durchführt. Eine derartige Herangehensweise ist generell nachteilig zum Erhalt von Polyisocyanaten mit enger Molekulargewichtsverteilung.

US 2011/0218314 beansprucht ein kontinuierliches Verfahren zur katalytischen Oligomerisierung von Isocyanaten unter Bildung von Carbamat- (Urethan)- bzw. Allophanatgruppen. Als Katalysatoren werden quaternäre Ammoniumhydroxide verwendet. Es wird auf einen extrem hohen Wärmeaustauschkoeffizienten verwiesen zur besseren Temperaturkontrolle innerhalb des Reaktors und einer damit einhergehenden besseren Kontrolle der Oligomerisierungsreaktion. Nachteilig bei den hier verwendeten Apparaten mit derartig hohen Wärmeaustauschkoeffizienten sind einerseits der hohe Druckverlust und andererseits die extrem ungünstige Geometrie, die eine hohe Anfälligkeit gegenüber gerade in der Isocyanatchemie häufig auftretenden Phänomenen wie spontaner Bildung von Gelteilchen, Viskositätserhöhungen etc. nach sich zieht.

Der hier vorliegenden Erfindung lag die Aufgabe zugrunde, ein Verfahren zur kontinuierlichen Herstellung von Polyisocyanaten zur Verfügung zu stellen, das nicht mit den vorgenannten Nachteilen behaftet ist: die Katalysatoren sollten nicht bzw. im Vergleich mit Verfahren des Standes der Technik weniger zur Zersetzung unter Bildung störender Nebenkomponenten neigen, die sich in den Verfahrensprodukten anreichern können. Sie sollen auch bei Einsatz in verdünnter alkoholischer Lösung zu batch-typischen Produkten führen, d.h. das molare Verhältnis der reinen Isocyanat-Oligomeren (Isocyanurate und/oder Iminooxadiazindione sowie in untergeordnetem Ausmaß auch Uretdione) zu den Produkten aus NCO-OH-Reaktionen (Urethane und Allophanate) sollte sich im aus batch-Experimenten bekannten Rahmen bewegen. Die Verfahrensprodukte sollten bei gegenüber der diskontinuierlichen Reaktionsführung ähnlichem Umsetzungsgrad des Monomers eine ähnliche Molmassenverteilung und damit einhergehend eine ähnliche Viskosität und einen ähnlichen NCO-Gehalt aufweisen.

Dies ist durch die Bereitstellung des erfmdungsgemäßen Verfahrens gelungen.

Gegenstand der Erfindung ist ein Verfahren zur kontinuierlichen Herstellung von oligomeren, bzw. polymeren Isocyanaten durch katalytische Modifizierung monomerer Di- und oder Triisocyanate, dadurch gekennzeichnet, dass mindestens eine Isocyanatkomponente und mindestens eine Katalysatorkomponente kontinuierlich in einem Reaktionsapparat zusammengeführt werden und als Reaktionsmischung durch den Reaktionsapparat geführt werden, wobei die Verweilzeitverteilung nach dem Dispersionsmodell durch Bo (Bodenstein-Zahl) oberhalb 40, bevorzugt oberhalb 60 und ganz besonders bevorzugt oberhalb 80 charakterisiert ist.

Keiner der eingangs genannten Schriften des Standes der Technik ist zu entnehmen, dass bei einer Reaktionsführung in kontinuierlich betriebenen prozessintensiven Apparaten mit vergleichbaren oder niedrigeren Katalysatorkonzentrationen als im Batch-Prozess Produkte mit ähnlichen Viskositäten und NCO-Gehalten ohne Umsatzeinbuße hergestellt werden können.

Kontinuierlich geführte Reaktionen im Sinne der Erfindung sind solche, bei denen der Zulauf der Edukte in den Reaktor und der Austrag der Produkte aus dem Reaktor gleichzeitig, aber räumlich getrennt stattfinden. Bei diskontinuierlicher Reaktion läuft demgegenüber die Reaktionsfolge - Zulauf der Edukte, chemische Umsetzung und Austrag der Produkte - zeitlich nacheinander ab.

Die kontinuierliche Verfahrensweise ist von wirtschaftlichem Vorteil, da Zeiten, in denen der Reaktor nicht zur Durchführung der Reaktion infolge von Befüllungs- und Entleerungsprozessen zur Verfügung steht und lange Reaktionszeiten infolge sicherheitstechnischer Vorgaben, reaktorspezifischer Wärmetauschleistungen als auch Aufheiz- und Abkühlungsprozesse, wie sie bei Batch-Verfahren auftreten, vermieden werden.

Die kontinuierliche Umsetzung erfolgt in einer bevorzugten Ausführungsform unter Verwendung einer Verweilstrecke im Druckbereich ≤30 bar, bevorzugt ≤10 bar, besonders bevorzugt im Bereich ≤4 bar.

Die Dosiergeschwindigkeiten aller Komponenten hängen in erster Linie von den gewünschten Verweilzeiten bzw. zu erreichenden Umsätzen ab. Die Verweilzeit wird unter anderem über die Volumenströme und das Volumen der Reaktionszone eingestellt. Je höher die maximale Reaktionstemperatur, desto kürzer sollte die Verweilzeit sein. In der Regel liegen die Verweilzeiten in der Reaktionszone zwischen 20 Sekunden (20 sek) und 120 Minuten (120 min), bevorzugt zwischen 90 sek und 90 min, ganz besonders bevorzugt zwischen 5 min und 60 min.

Der Reaktionsverlauf wird vorteilhaft durch verschiedene Messeinrichtungen verfolgt. Dazu geeignet sind insbesondere Einrichtungen zur Messung der Temperatur, der Viskosität, des Brechungsindex und/oder der Wärmeleitfähigkeit in strömenden Medien und/oder zur Messung von Infrarot- und/oder Nahinfrarotspektren.

Bevorzugt weisen die erfindungsgemäß zu verwendenden Reaktionsstrecken eine geeignete Wärmeübertragungsleistung auf, welche durch die spezifische Wärmeübertragungsrate in W/(K m³) gekennzeichnet ist, also Wärmeübertrag pro Kelvin Temperaturdifferenz zum Wärmeträgermedium bezogen auf das freie Volumen des Reaktors. In einer bevorzugten Ausführungsform sind die erfindungsgemäß einzusetzenden Reaktionsstrecken demzufolge dadurch charakterisiert, dass sie durch ihren Aufbau im erfindungsgemäßen Verfahren a) einen maximalen Druckverlust von 0 bis 30 bar, bevorzugt 0 bis 10 bar und insbesondere bevorzugt 0 bis 4 bar ermöglichen und b) eine Wärmeübertragungsrate oberhalb 10 kW/(K m³), bevorzugt oberhalb 50 kW/(K m³) und besonders bevorzugt oberhalb 100 kW/(K m³) ermöglichen.

Hier bietet sich beispielsweise die Verwendung der Mikroreaktionstechnik (µ-Reaktionstechnik) unter Einsatz von Mikroreaktoren an. Die verwendete Bezeichnung "Mikroreaktor" steht dabei stellvertretend für mikrostrukturierte, vorzugsweise kontinuierlich betriebene Reaktoren, die unter der Bezeichnung Mikroreaktor, Minireaktor, Mikrowärmetauscher, Minimischer oder Mikromischer bekannt sind. Beispiele sind Mikroreaktoren, Mikrowärmetauscher, T- und Y-Mischer sowie Mikromischer verschiedenster Firmen (z.B. Ehrfeld Mikrotechnik BTS GmbH, Institut für Mikrotechnik Mainz GmbH, Siemens AG, CPC-Cellulare Process Chemistry Systems GmbH, und anderen), wie sie dem Fachmann allgemein bekannt sind, wobei ein "Mikroreaktor" im Sinne der vorliegenden Erfindung üblicherweise charakteristische, bestimmende innere Abmessungen von bis zu 1 mm aufweist und statische Mischeinbauten enthalten kann.

Ebenso geeignet sind Intensivwärmetauscher, wie z.B. CSE-XR-Typen der Firma Fluitec, sofern sie die oben genannten Eigenschaften hinsichtlich ihrer Wärmeübertragungseigenschaften erfüllen können. Ebenfalls denkbar sind hier Verknüpfungen von Mikroreaktoren mit anderen größer strukturierten Wärmetauschern, wie z.B. der Firma Fluitec oder Sulzer. Wesentliches Merkmal bei diesen Verknüpfungen ist die Anordnung der einzelnen Reaktortypen entsprechend der jeweilig erwarteten, notwendigen Wärmeübertragungsleistung der einzelnen Apparate unter Berücksichtigung der auftretenden Viskositäten bzw. Druckverlusten.

Damit Polyisocyanate mit den gewünschten Produkteigenschaften hergestellt werden können, ist eine enge Verweilzeitverteilung im Reaktorsystem von entscheidender Bedeutung. Die Bodenstein-Zahl Bo, als Maß für die Breite der Verweilzeitverteilung nach dem Dispersionsmodell, liegt oberhalb 40, bevorzugt oberhalb 60 und ganz besonders bevorzugt oberhalb 80.

Erreicht wird die enge Verweilzeitverteilung üblicherweise durch die Verwendung von statischen Mischelementen oder von µ-Reaktoren, wie oben beschrieben. Typischerweise erfüllen ebenso Intensivwärmetauscher wie z.B. der CSE-XR-Typ diese Anforderung hinreichend.

Die Komponenten werden dem Reaktor in der Regel in getrennten Eduktströmen zudosiert. Bei mehr als zwei Eduktströmen können diese auch gebündelt zugeführt werden. Es ist auch möglich, diesem Produktstrom zusätzlich Katalysatoren und/oder Zuschlagstoffe wie Verlaufshilfsmittel, Stabilisatoren oder Haftvermittler zuzufügen.

Es ist möglich, Stoffströme in unterschiedlichen Anteilen an verschiedenen Stellen dem Reaktor zuzuführen, um auf diese Weise gezielt Konzentrationsgradienten einzustellen, z.B. um die Vollständigkeit der Reaktion herbeizuführen. Die Eintrittsstelle der Stoffströme in der Reihenfolge kann variabel und zeitlich versetzt gehandhabt werden. Zur Vorreaktion und / oder Vervollständigung der Reaktion können mehrere Reaktoren kombiniert werden. Zum Abstoppen der Reaktion bei gewünschtem Umsetzungsgrad können im Anschluss an die Reaktionsstrecke weitere Komponenten zugeführt werden.

Einzelne oder alle Stoffströme können vor der Zusammenführung durch einen Wärmetauscher temperiert werden, d.h. auf eine Temperatur von -20°C und +200°C, bevorzugt +20°C bis +180°C, besonders bevorzugt +40°C bis +120°C gebracht werden. Anschließend werden sie mit einem Intensiv-Mischer vermischt und durch einen oder mehrere Reaktoren gefördert. Bevorzugt erfolgt die Zusammenführung der Komponenten unter Verwendung von Mischelementen, die eine intensive Vermischung der Reaktionspartner bewirken. Vorteilhaft ist der Einsatz eines Intensiv-Mischers (µ-Mischer), mit dessen Hilfe die Reaktionslösungen trotz unterschiedlicher Volumenströme und ggf. auch differierender Viskosität sehr schnell miteinander vermischt werden, wodurch ein möglicher radialer Konzentrationsgradient vermieden wird. Nach der Zusammenführung/Mischung der Reaktionspartner werden diese durch den Reaktionsapparat, der ggf. weitere Mischelemente enthält, gefördert. Weitere Mischelemente entlang der Reaktionsstrecke führen zu einer bevorzugten engeren Verweilzeitverteilung. Der Reaktionsapparat ist dadurch gekennzeichnet, dass er ein Verweilvolumen von mindestens 20 sek bis 120 min zur Verfügung stellt. Alle Reaktoren sind vorteilhaft mit einer Kühl- und / oder Heizvorrichtung z.B. einem Mantel, durch den eine temperierte Wärmeträgerflüssigkeit geleitet wird, versehen.

Die Verwendung mehrerer unabhängig temperierbarer Heiz/Kühlzonen ermöglicht es zum Beispiel, das strömende Reaktionsgemisch zu Beginn der Reaktion, also kurz nach dem Vermischen, zu kühlen und freiwerdende Reaktionswärme abzuführen und gegen Ende der Reaktion, also kurz vor dem Austrag aus dem Reaktor, zu heizen, so dass für den gewünschten Umsatz notwendige Verweilvolumen möglichst klein gehalten werden kann. Die Kühl- und Heizmitteltemperatur kann dabei zwischen -20 und +250°C liegen.

Mit dem erfindungsgemäßen Modifizierverfahren ist daher eine verbesserte Methode zur kontinuierlichen Herstellung von Isocyanattrimeren zugänglich geworden.

Das erfindungsgemäße Verfahren kann im Temperaturbereich von 0°C und + 200°C, bevorzugt 20°C bis 180°C, besonders bevorzugt 40°C bis 120°C durchgeführt werden, wobei bevorzugt über den gesamten Umsatzbereich eine konstante Reaktionstemperatur realisiert wird, und bei beliebigen Umsetzungsgraden, bevorzugt nachdem 5 bis 80 %, besonders bevorzugt 10 bis 60 %, ganz besonders bevorzugt 10 bis 45 % des eingesetzten monomeren Diisocyanates umgesetzt wurden, unterbrochen werden. Das nicht umgesetzte Monomer wird anschließend wiedergewonnen und im erfindungsgemäßen Verfahren, mit oder ohne Zusatz von frischem Monomer und/oder anderen Zuschlagstoffen und Additiven, erneut eingesetzt.

Als Katalysatoren kommen prinzipiell alle für diesen Zweck vorbeschriebenen Verbindungen des Standes der Technik als solche oder in, ggf. auch stark verdünnter, Lösung in Frage. Besonders geeignet erscheinen salzartig aufgebaute Substanzen mit Kationen, die für eine gute Löslichkeit im Isocyanatmedium sorgen, besonders Tetraorganyl-ammonium und -phosphonium, und Anionen, die sich durch H⁺-Abstraktion aus schwachen Säuren wie z.B. Carbonsäuren, Phenolen, Alkoholen, Wasser, HF etc. ableiten. Fluoride, Hydrogendifluoride, Dihydrogentrifluoride und höhere Polyfluoride,wie auch die Anionen spezieller, fluorierte Alkylgruppen tragender Carbonsäuren, führen verstärkt zur Bildung hoch Iminooxadiazindiongruppen-haltiger Isocyanat-Trimerer.

Geeignete Trimerisierungskatalysatoren sind weiterhin alkoholische Lösungen von Alkali- und Erdalkali-oxiden, -hydroxiden, -alkoholaten sowie -phenolaten. Weiterhin eignen sich alkoholische Lösungen von Metallsalzen von Carbonsäuren, beispielsweise Kaliumacetat, Natriumbenzoat, Natriumacetat und Kaliumformiat sowie ferner tertiäre Phosphine, insbesondere solche, die das Phosphoratom als Teil eines Cyclus' enthalten.

Bevorzugt können für das erfindungsgemäße Verfahren folgende Katalysatoren eingesetzt werden: Quaternäre Ammoniumhydroxide, vorzugsweise N,N,N-Trimethyl-N-benzylammoniumhydroxid und N,N,N-Trimethyl-N-(2-hydroxypropyl)-ammoniumhydroxid. Quaternäre Ammonium- sowie Phosphonium-fluoride, - difluoride, -trifluoride und höhere -polyfluoride, wie sie durch einfaches Abmischen von quaternären Ammonium- sowie Phosphonium-fluoriden oder auch -hydroxiden mit entsprechenden Anteilen, optional in Alkoholen oder Wasser vorgelöster, HF bereitet werden können.

Die Art des Trimerisierungskatalysators spielt für die Durchführung des erfindungsgemäßen Verfahrens insofern eine Rolle, als sie die Produktselektivität hinsichtlich der Bildung von überwiegend Uretdion-, Isocyanurat- und/oder Iminooxadiazindiongruppen enthaltenden Produkten wesentlich beeinflusst. Insbesondere können durch Wahl des Katalysators und der Reaktionsbedingungen optional geringe, aber durchaus gezielt einstellbare Anteile an Uretdiongruppen tragenden Oligomerbestandteilen in ansonsten überwiegend aus den beiden Isocyanat-Trimerstrukturen aufgebauten Oligomerenmischungen implementiert werden, was ausdrücklich im Umfang der erfindungsgemäßen Verfahrensdurchführung eingeschlossen ist.

Der Katalysatorbedarf im erfindungsgemäßen Verfahren liegt nicht über dem in der Bulk-Modifizierung des Standes der Technik (Diskonti-"batch-"Verfahren) beobachteten und ist häufig sogar noch niedriger. Dies ist besonders überraschend und konnte nicht erwartet werden.

Der Katalysator kann beispielweise in einem Anteil zwischen 1 mol-ppm und 1 mol-%, bevorzugt zwischen 5 mol-ppm und 0,1 mol-%, bezogen auf die Masse an eingesetztem Monomer, eingesetzt werden. Die Katalysatormenge ist ein weiterer Parameter, um die benötigte Verweilzeit einzustellen. Im erfindungsgemäßen Verfahren wird auch bei hohen Katalysatorkonzentrationen problemlos eine gute Temperaturkontrolle erreicht, so dass deutlich kürzere Verweilzeiten als im etablierten Diskonti-Batch-Verfahren möglich sind.

Zur Katalysatordeaktivierung bieten sich prinzipiell eine ganze Reihe vorbeschriebener Methoden des Standes der Technik an, wie z.B. die Zugabe (bzgl. des Katalysatoreinsatzes unter- oder über-) stöchiometrischer Mengen an Säuren (z.B. HCl, Phosphor oder Schwefel enthaltender Säuren, nicht jedoch HF) oder Säurederivaten (z.B. Säurechloride, saure Ester Phosphor oder Schwefel enthaltender Säuren, etc.). Zur Katalysatordeaktivierung geeignet sind weiterhin Alkylierungsmittel im Falle der Verwendung von dreiwertigen Phosphorverbindungen als Katalysatoren, adsorptive Bindung des Katalysators und anschließende Abtrennung durch Filtration, thermische Desaktivierung, destillative Rückgewinnung, bevorzugt gemeinsam mit dem zurückzuführenden, nicht umgesetzten Monomer, Extraktion usw..

Die im erfindungsgemäßen Verfahren resultierenden monomerenarmen Isocyanattrimeren weisen mindestens das gleiche, hohe Qualitätsniveau auf, wie die Produkte, die nach vorbeschriebenen Verfahren des Standes der Technik erhalten werden und sind von ihnen analytisch nicht unterscheidbar. Der bei Verwendung von Katalysatoren mit quaternären Ammoniumsalzen auf Basis, insbes. kurzkettiger, Alkyl- oder Aralkylsubstituenten am Stickstoffatom mitunter wahrzunehmende Amingeruch der Verfahrensprodukte ist deutlich reduziert bzw. eliminiert. Die Kontamination des Recyclat-Monomers mit P-haltigen Substanzen im Falle der Katalyse mit Phosphonium-kationen aufweisenden Katalysatoren ist ebenfalls signifikant reduziert.

Handelt es sich bei den Verfahrensprodukten um Polyisocyanate ("Trimerisate") vom Iminooxadiazindiontyp, liegt der Anteil an Iminooxadiazindiongruppen, bezogen auf die Gesamtmenge an durch die Modifizierungsreaktion aus vorher freien NCO-Gruppen gebildeten Folgeprodukten, bevorzugt oberhalb 30 mol-%, besonders bevorzugt oberhalb 40 mol-%, wobei die Differenz zu 100% im wesentlichen Isocyanuratgruppen zuzuordnen ist. Der Uretdionanteil dieser speziellen Verfahrensprodukte liegt unterhalb 20 mol-%, bevozugt unterhalb 10 mol-%.

Zur Durchführung des erfindungsgemäßen Verfahrens können prinzipiell alle bekannten (Di)Isocyanate des Standes der Technik einzeln oder in beliebigen Abmischungen untereinander eingesetzt werden.

Insbesondere seien genannt: 1,5-Pentandiisocyanat, Hexamethylendiisocyanat (HDI), 2-Methylpentan-1,5-diisocyanat, 2,4,4-Trimethyl-1,6-hexandiisocyanat, 2,2,4-Trimethyl-1,6-hexandiisocyanat, 4-Isocyanatomethyl-1,8-octandiisocyanat, 3(4)-Isocyanatomethyl-1 - methylcyclohexylisocyanat (IMCI), Isophorondiisocyanat (IPDI), 1,3- sowie 1,4-Bis(isocyanatomethyl)benzen (XDI), 1,3- sowie 1,4-Bis(isocyanatomethyl)cyclohexan (H6XDI).

Weiterhin können aromatische Isocyanate wie 2,4- sowie 2,6-Toluylendiisocyanat (TDI), Bis(4-isocyanatophenyl)methan (4,4'MDI), 4-Isocyanatophenyl-2-isocyanatophenylmethan (2,4'MDI) sowie mehrkernige Produkte, die durch Formaldehyd-Anilin-Polykondensation und anschließende Überführung der resultierenden (Poly)amine in die entsprechenden (Poly)isocyanate zugänglich sind (Polymer-MDI) im erfindungsgemäßen Verfahren eingesetzt werden.

Bevorzugt sind aliphatische Di- und/oder Triisocyanate, besonders bevorzugt aliphatische Diisocyanate, ganz besonders bevorzugt Hexamethylendiisocyanat (HDI), 2-Methylpentan-1,5-diisocyanat, 2,4,4-Trimethyl-1,6-hexandiisocyanat, 2,2,4-Trimethyl-1,6-hexandiisocyanat, 4-Isocyanatomethyl-1,8-octandiisocyanat, noch mehr bevorzugt ist HDI.

Hierbei ist es belanglos, nach welchen Verfahren die vorstehend genannten Di- bzw. (Poly)isocyanate generiert werden, d.h. mit oder ohne Verwendung von Phosgen.

Die nach dem erfindungsgemäßen Verfahren erhältlichen Produkte bzw. Produktgemische stellen mithin vielseitig verwendbare Ausgangsmaterialien zur Herstellung von, ggf. geschäumten, Kunststoffen sowie Lacken, Beschichtungsmitteln, Klebstoffen und Zuschlagstoffen dar. Insbesondere zur Herstellung von, ggf. wasserdispergierbaren Ein- und Zweikomponenten-Polyurethanlacken eignen sie sich, ggf. in NCO-blockierter Form, aufgrund ihrer im Vergleich zu (überwiegend) Isocyanurat-Polyisocyanat basierenden Produkten verringerten Lösungs- sowie Schmelzviskosität bei ansonsten gleich hohem bzw. verbessertem Eigenschaftsprofil. So sind die erfindungsgemäßen Verfahrensprodukte auf HDI-Basis auch in hoher Verdünnung in Lacklösungsmitteln stabiler gegen das Auftreten von Ausflockungen bzw. Trübungen, als entsprechende Produkte auf Isocyanuratbasis.

Sie können rein oder in Verbindung mit anderen Isocyanatderivaten des Standes der Technik, wie z.B. Uretdion-, Biuret-, Allophanat-, Isocyanurat- und/oder Urethan-Gruppen enthaltenden Polyisocyanaten, deren freie NCO-Gruppen ggf. mit Blockierungsmitteln desaktiviert wurden, eingesetzt werden.

Die nachfolgenden Vergleichsbeispiele und Beispiele sollen die Erfindung näher erläutern, ohne sie jedoch einzuschränken.

Alle Mengenangaben beziehen sich, soweit nicht anders vermerkt, auf die Masse.

Die Ermittlung des NCO-Gehaltes der in den Beispielen und Vergleichsbeispielen beschriebenen Harze erfolgte durch Titration gemäß DIN EN ISO 11 909.

Der Phosphorgehalt wurde durch Röntgenfluoreszenzanalyse (RFA) bestimmt.

Die Bestimmung der Restmonomergehalte erfolgte gaschromatographisch gemäß DIN EN ISO 10283.

Die dynamischen Viskositäten wurden bei 23 °C mit dem Viskosimeter VT 550 der Fa. Haake bestimmt. Durch Messungen bei unterschiedlichen Schergeschwindigkeiten wurde sichergestellt, dass das Fließverhalten der beschriebenen erfindungsgemäßen Polyisocyanatmischungen wie auch das der Vergleichsprodukte dem idealer Newtonscher Flüssigkeiten entspricht. Die Angabe der Schergeschwindigkeit kann deshalb entfallen.

Mol-% Angaben wurden NMR-spektroskopisch ermittelt und beziehen sich immer, so nicht anders ausgewiesen, auf die Summe der in der Modifizierungsreaktion neu gebildeten NCO-Folgeprodukte. Die Messungen erfolgten auf den Geräten DPX 400 bzw. DRX 700 der Fa. Bruker an ca. 5 %igen (¹H-NMR) bzw. ca. 50 %igen (¹³C-NMR) Proben in trockenem C₆D₆ bei einer Frequenz von 400 bzw. 700 MHz (¹H-NMR) oder 100 bzw. 176 MHz (¹³C-NMR). Als Referenz für die ppm-Skala wurden geringe Mengen von Tetramethylsilan im Lösungsmittel mit 0 ppm ¹H-NMR-chem. Verschiebung heran gezogen. Alternativ wurde auf das Signal des im Lösungsmittel enthaltenen C₆D₅H referenziert: 7,15 ppm ¹H-NMR-chem. Verschiebung, 128,02 ppm ¹³C-NMR-chem. Verschiebung. Daten für die chemische Verschiebung der in Frage kommenden Verbindungen wurden der Literatur entnommen (vgl. D. Wendisch, H. Reiff und D. Dieterich, Die Angewandte Makromolekulare Chemie 141, 1986, 173-183 und darin zit. Lit. sowie EP 896 009.

Alle Reaktionen wurden, wenn nicht anders angegeben, unter einer Stickstoffatmosphäre durchgeführt.

Die verwendeten Diisocyanate sind Produkte der Bayer MaterialScience AG, D-51368 Leverkusen, alle anderen kommerziell zugänglichen Chemikalien wurden von der Fa. Aldrich, D-82018 Taufkirchen, bezogen. Die Herstellung der Hydrogenpolyfluoridkatalysatoren ist literaturbekannt und wird u.a. in EP 962 454 beschrieben.

### Beispiel 1 Vergleichsbeispiel

In einem doppelwandigen, durch einen externen Kreislauf auf 60°C temperierten Planschliffgefäß mit Rührer, an eine Inertgasanlage (Stickstoff/Vakuum) angeschlossenem Rückflusskühler und Thermometer wurden 1000 g HDI vorgelegt und durch einstündiges Rühren im Vakuum (0,1 mbar) von gelösten Gasen befreit. Nach Belüften mit Stickstoff wurden 507 mg einer 70%igen isopropanolischen Lösung von Tetrabutylphosphoniumhydrogendifluorid portionsweise so dosiert, dass die Temperatur der Reaktionsmischung 65°C nicht überschritt. Nachdem ca. 1 mol NCO Gruppen umgesetzt waren, wurde der Katalysator durch Zugabe einer zum Katalysator äquivalenten Menge an p-Toluolsulfonsäure (als 40%ige Lösung in Isopropanol, "Stopperlösung") deaktiviert, weitere 30 min bei Reaktionstemperatur nachgerührt und anschließend aufgearbeitet. Die Aufarbeitung der Rohlösung, deren bei 25°C gemessener Brechungsindex bei der Frequenz des Lichtes der D-Linie des Na-Emissionsspektrums (im weiteren Text n_{D}²⁵) 1,4602 betrug, erfolgte durch Vakuumdestillation in einem Dünnschichtverdampfer, Typ Kurzwegverdampfer (KWV), mit vorgeschaltetem Vorverdampfer (W) (Destillationsdaten: Druck: 0,08 +/- 0,04 mbar, VV-Temperatur: 120°C, KWV-Temp.: 140°C), wobei nicht umgesetztes Monomer als Destillat und das monomerenarme Polyisocyanatharz als Sumpfprodukt separiert wurden (Startdurchlauf, Beispiel 1-A). Das Polyisocyanatharz wurde separiert und das Destillat in einer zweiten Planschliff-Rührapparatur, die identisch zur ersten aufgebaut ist, gesammelt und mit frisch entgastem HDI auf die Ausgangsmenge (1000 g) ausgefüllt. Anschließend wurde erneut katalysiert und verfahren wie eingangs beschrieben. Diese Verfahrensweise wurde unter Variation von Katalysatormenge, Umsatz und Reaktionszeit insgesamt fünfmal wiederholt (Details s. Tabelle 1). Aus der Analyse der Phosphorgehalte der erhaltenen Polyisocyanatharze und des am Ende der Versuchsserie verbleibenden Recyclatmonomers wurde die Phosphorbilanz ermittelt. Sie beläuft sich bei summarisch 92 % Wiederfindung auf 79 % des gefundenen Phosphors in den Harzen und 21 % im letzten Destillat. Die Daten der in den Versuchen 1-B bis 1-F erhaltenen Polyisocyanatharze sind ebenfalls Tabelle 1 zu entnehmen.

**Tabelle 1**

| **Bsp. 1** | Bu₄P⁺ HF₂]⁻ Lösung.^{(a)} [mg] | Reaktionszeit [hh:mm] | n_{D}^{25 (b)} | Harzmenge [g] | Harz-NCO | Harzviskosität [mPas/23°C] | Isocyanurate^{(c)} | Iminooxadiazindione^{(c)} | Uretdione^{(c)} |
|---|---|---|---|---|---|---|---|---|---|
| **A** | 507 | 00:34 | 1,4602 | 195 | 23,6% | 694 | 50,5 | 44,3 | 5,2 |
| **B** | 340 | 00:22 | 1,4598 | 198 | 23,5% | 684 | 47,1 | 49,4 | 3,5 |
| **C** | 214 | 00:30 | 1,4595 | 162 | 23,6% | 680 | 46,3 | 48,6 | 5,1 |
| **D** | 134 | 00:48 | 1,4590 | 159 | 23,9% | 710 | 65,3 | 32,8 | 1,9 |
| **E** | 611 | 01:12 | 1,4653 | 300 | 22,8% | 1375 | 56,5 | 38,0 | 5,5 |
| **F** | 714 | 00:38 | 1,4689 | 391 | 21,5% | 1940 | 44,0 | 51,3 | 4,7 |

| | | | | | | | | | |
|---|---|---|---|---|---|---|---|---|---|
| ^{(a)} 70%ig in iPrOH; ^{(b)} Brechungsindex der Reaktionsmischung nach Einwirkung des Stoppers vor der Destillation, ^{(c)} mol-% lt. NMR, bezogen auf die Summe der in der Modifizierungsreaktion gebildeten NCO-Folgeprodukte | | | | | | | | | |

### Beispiel 2 Vergleichsbeispiel

Es wurde verfahren wie in Beispiel 1 angegeben, mit dem Unterschied, dass als Katalysator jeweils eine 0,5%ige Lösung von Benzyltrimethylammoniumhydroxid (BzMe₃N⁺ OH⁻) in 2-Ethylhexanol (Bspp. 2-A bis 2-F) bzw. 2-Ethyl-1,3-hexandiol (Bspp. 2-G bis 2-L, Mengen s. Tabelle 2) und als Stopper die stöchiometrische Menge Dibutylphosphat (Mengen s. Tabelle 2) zum Einsatz kam. Die analytischen Daten der in den Beispielen 2-A bis 2-L erhaltenen Polyisocyanatharze, die unmittelbar nach der Herstellung einen deutlich wahrnehmbaren Amingeruch aufwiesen, sind ebenfalls Tabelle 2 zu entnehmen.

**Tabelle 2**

| **Bsp. 2** | BzMe₃N⁺ OH⁻ - Lsg. [g] | Dibutylphosphat [g] | Reaktionszeit [hh:mm] | n_{D}^{25 (a)} | Harzmenge [g] | Harz-NCO | Harz-Viskosität [mPas/23°C] | Isocyanurate^{(b)} | Iminooxadiazindione^{(b)} | Allophanate^{(b)} | Uretdione^{(b)} |
|---|---|---|---|---|---|---|---|---|---|---|---|
| **A** | 12,05^{(c)} | 0,08 | 2:22 | 1,4617 | 238 | 22,4% | 1040 | 81,4 | 3,9 | 14,3 | 0,4 |
| **B** | 15,00^{(c)} | 0,09 | 2:59 | 1,4648 | 298 | 21,9% | 1400 | 83,1 | 2,8 | 13,8 | 0,3 |
| **C** | 15,20^{(C)} | 0,10 | 2:19 | 1,4702 | 402 | 21,5% | 2240 | 85,2 | 2,1 | 12,5 | 0,2 |
| **D** | 15,18^{(c)} | 0,10 | 2:42 | 1,4753 | 497 | 21,0% | 4340 | 89,2 | 1,4 | 9,1 | 0,3 |
| **E** | 20,30^{(c)} | 0,12 | 2:30 | 1,4837 | 604 | 19,6% | 11000 | 89,4 | 1,8 | 8,6 | 0,2 |
| **F** | 25.05^{(c)} | 0,16 | 3:01 | 1,4911 | 701 | 18,2% | 40000 | 89,7 | 1,3 | 8,6 | 0,4 |
| **G** | 10,05^{(d)} | 0,07 | 0:04 | 1,4611 | 198 | 22,0% | 2570 | 70,9 | 1,4 | 26,4 | 1,3 |
| **H** | 10,05^{(d)} | 0,07 | 0:50 | 1,4646 | 296 | 21,9% | 2940 | 78,1 | 1,2 | 18,8 | 1,9 |
| **I** | 7,63^{(d)} | 0,06 | 1:30 | 1,4685 | 405 | 21,8% | 3610 | 88,2 | 1,5 | 9,4 | 0,9 |
| **J** | 9,93^{(d)} | 0,07 | 2:09 | 1,4741 | 506 | 21,0% | 6300 | 86,3 | 1,4 | 11,5 | 0,8 |
| **K** | 13,10^{(d)} | 0,08 | 3:46 | 1,4828 | 598 | 20,0% | 18000 | 87,4 | 1,9 | 9,9 | 0,9 |
| **L** | 20,00^{(d)} | 0,13 | 4:24 | 1,4903 | 694 | 19,2% | 59000 | 86,0 | 1,6 | 12,0 | 0,4 |

| | | | | | | | | | | | |
|---|---|---|---|---|---|---|---|---|---|---|---|
| ^{(a)} Brechungsindex der Reaktionsmischung nach Einwirkung des Stoppers vor der Destillation, ^{(b)} mol-% lt. NMR, bezogen auf die Summe der in der Modifizierungsreaktion gebildeten NCO-Folgeprodukte, ^{(c)} 0,5%ig in 2-Ethylhexanol gelöst; ^{(d)} 0,5%ig in 2-Ethyl-1,3-hexandiol gelöst | | | | | | | | | | | |

### Beispiel 3 erfindungsgemäß

Mit analog Beispiel 1 vorbehandeltem (entgastem) HDI und den unter Beispiel 1 eingesetzten Katalysator- sowie Stopperlösungen wurde in einem kontinuierlich betriebenen Aufbau wie im Folgenden beschrieben verfahren.

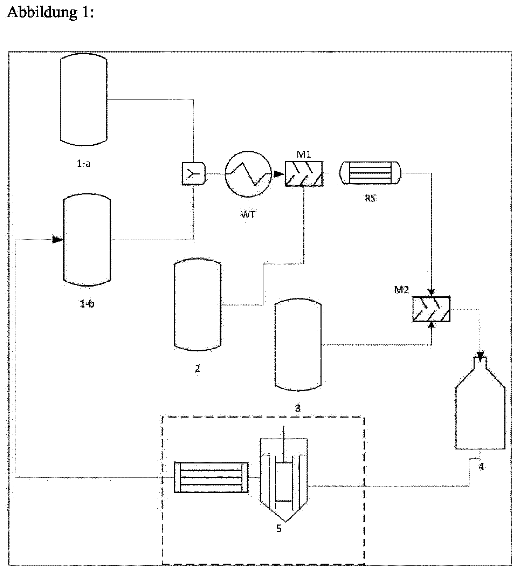

Abbildung 1 zeigt schematisch einen Aufbau zur Durchführung des erfindungsgemäßen Verfahrens. Es sind vier Vorlagen 1-a, 1-b, 2 und 3 vorhanden, aus denen die Edukte der Reaktionsstrecke getrennt zugeführt wurden.

Die Vorlagen 1-a und 1-b enthielten das umzusetzende, monomere (Di)Isocyanat (1a das neu dem Prozeß zugeführte Monomer, 1b das mittels Destillation zurückgewonnene). Vorlage 2 enthielt den Katalysator. Die Ströme 1 und 2 wurden zunächst einem ersten Mischelement (M1) zugeführt. Die Stoffströme wurden vor dem Eintritt in das erste Mischelement über Wärmetauscher (WT) auf die Solltemperatur gebracht.

Beim ersten Mischelement handelte es sich um einen µ-strukturierten Kaskadenmischer der Fa. Ehrfeld Mikrotechnik BTS GmbH.

Nach intensiver Vermischung der Komponenten wurde der Stoffstrom in die Reaktionsstrecke (RS) geführt, gebildet im vorliegenden Beispiel durch mehrere hintereinander geschaltete Wärmetauscher der Fa. Fluitec. Auch können hier alternativ statische Mischelemente wie z.B. Kenics oder SMX eingesetzt werden. Die Temperatur des Reaktionsgemisches wurde durch intensive Wärmeübertragung auf der Solltemperatur (60 +/- 0,5 °C) gehalten.

Nach einer definierten Verweilstrecke wurde dem Reaktionsmedium eine weitere Komponente aus einer Vorlage 3 (Stopperlösung) über ein weiteres Mischelement (M2) zugeführt. Das Reaktionsgemisch durchlief eine definierte Verweilstrecke (Bo ca. 90) bestehend aus Wärmeübertragungselementen bevor es den Zwischenprodukt-Behälter (4) erreichte und von dort in die (destillative) Monomerabtrennung, bestehend aus einem Vorverdampfer und einem Kurzwegverdampfer (5) gefördert wurde. Das dabei destillativ anfallende, nicht umgesetzte Monomer wurde nach 1b zurück geführt (s.o.) während das monomerenarme Polyisocyanat ausgeschleust wurde.

Die Destillation erfolgte in der gleichen Apparatur wie in Beispiel 1 beschrieben.

Es wurden über 24 h bei einem gemittelten Verbrauch an Katalysatorlösung analog Bsp. 1 um 300 mg/kg_{HDI} (Stopperlösung analog Bsp. 1: 305 mg/kg_{HDI}) mit hoher Konstanz im Mittel 210 g_{Harz}/kg_{HDI} eines Polyisocyanatharzes mit folgenden Daten erhalten (Beispiel 3a):
NCO-Gehalt: 23,5 %
Viskosität: 710 mPas/23°C
Iminooxadiazindione: 49 mol-%*
Isocyanurate: 47 mol-%*
Uretdione: 4 mol-%*
* = bezogen auf die Summe der in der Modifizierungsreaktion gebildeten NCO-Folgeprodukte

Anschließend wurde die Katalysatordosierung stufenweise auf minimal 105 mg/kg HDI abgesenkt. Bei dieser Einstellung und entsprechend angepasster Stopperdosierung (108 mg/kg_{HDI}) wurden weitere 24 h mit hoher Konstanz im Mittel 195 g_{Harz}/kg_{HDI} eines Polyisocyanatharzes mit folgenden Daten erhalten (Beispiel 3b):
NCO-Gehalt: 23,7 %
Viskosität: 710 mPas/23°C
Iminooxadiazindione: 45 mol-%*
Isocyanurate: 48 mol-%*
Uretdione: 7 mol-%*
* = bezogen auf die Summe der in der Modifizierungsreaktion gebildeten NCO-Folgeprodukte

Aus der Analyse der Phosphorgehalte der erhaltenen Polyisocyanatharze und des am Ende der Versuchsserie verbleibenden Recyclatmonomers wurde die Phosphorbilanz ermittelt. Sie beläuft sich bei summarisch 96 % Wiederfindung auf 98 % des gefundenen Phosphors in den Harzen und 2 % im letzten Destillat, was eine deutliche Verbesserung gegenüber dem in Vergleichsbeispiel 1 gefundenen Resultat darstellt.

Wie ein Vergleich des Ergebnisses des vorstehend genannten, erfindungsgemäßen Beispiels 3b mit dem in Vergleichsbeispiel 1d erhaltenen Resultat belegt, ist der Iminooxadiazindiongruppenanteil des Harzes aus dem erfindungsgemäßen Beispiel bei signifikant erniedrigter Katalysatormenge (relativ zum umgesetzten Monomer) und ansonsten gleichen Reaktionsbedingungen deutlich erhöht.

### Beispiel 4 erfindungsgemäß

Mit analog Beispiel 2 vorbehandeltem (entgastem) HDI und der unter Beispiel 2-A bis 2-F eingesetzten Katalysatorlösung sowie Dibutylphosphat als Stopper (als 10%ige Lösung in 2-Ethylhexanol) wurde in einer Apparatur wie in Beispiel 3 beschrieben über 24 h bei einem gemittelten Katalysatorverbrach um 4,5 g/kg_{HDI} (Stopperlösung entsprechend 285 mg/kg_{HDI}) mit hoher Konstanz ein Polyisocyanatharz mit folgenden Daten erhalten:
Harzausbeute (bezogen auf eingesetztes HDI): 21,8%
NCO-Gehalt: 23,2 %
Viskosität: 1250 mPas/23°C
Iminooxadiazindione: 3 mol-%*
Isocyanurate: 88 mol-%*
Uretdione: 3 mol-%
Allophanate: 6 mol-%
* = bezogen auf die Summe der in der Modifizierungsreaktion gebildeten NCO-Folgeprodukte

Auch unmittelbar nach Entnahme der Harze aus der Destillationsapparatur war kein Amingeruch wahrnehmbar.

### Beispiel 5 Vergleichsbeispiel

Mit analog Beispiel 1 vorbehandeltem (entgastem) HDI und den unter Beispiel 1 eingesetzten Katalysator- sowie Stopperlösungen wurde in einer Apparatur analog Beispiel 3 jedoch bei bewusst breiter gewählter Verweilzeitverteilung (Bo ca. 30, d.h. gegenüber Beispiel 3 um den Faktor 3 reduziert) die folgenden Daten erhalten:
Harzausbeute (bezogen auf eingesetztes HDI): 18,1%
NCO-Gehalt: 22,8 %
Viskosität: 1030 mPas/23°C
Iminooxadiazindione: 42 mol-%*
Isocyanurate: 55 mol-%*
Uretdione: 4 mol-%

### Beispiel 6 erfindungsgemäß

Mit analog Beispiel 3 eingesetzter Apparatur wurde bei gegenüber Beispiel 3a 6-facher Katalysatormenge (bezogen auf Monomer) und trotzdem sehr guter Temperaturkontrolle (Temperaturerhöhung <1 K) die Raumzeitausbeute auf das 5 fache erhöht und ein Polyisocyanatharz mit folgenden Daten erhalten:
Harzausbeute (bezogen auf eingesetztes HDI): 29,5 %
NCO-Gehalt: 22,3 %
Viskosität: 1120 mPas/23°C
Iminooxadiazindione: 48 mol-%*
Isocyanurate: 47 mol-%*
Uretdione: 5 mol-%
Der Brechungsindex der abgestoppten Rohware (n_{D}²⁵) vor der Destillation betrug im Mittel 1,4658.

Ein Vergleich der Beispiele 1-A bis 1-F sowie des Beispiels 6 mit den in EP 17998 angegebenen Beispielen zur HDI-Trimerisierung belegt einen signifikant niedrigeren NCO-Gehalt in den monomerenarmen Polyisocyanaten gemäß EP 17998 (20,9 bis 21,4 %) in Relation zum Monomerumsatz (indiziert durch den Brechungsindex der Rohware (n_{D}²⁵) - in EP 17998 zwischen 1,4658 und 1,4676).

Daraus wird sofort ersichtlich, dass nach der Lehre der EP 17998 nur Produkte mit deutlich breiterer Molekulargewichtsverteilung als im Batchversuch und im erfindungsgemäßen Verfahren zugänglich sind.

## Patentansprüche

1. Verfahren zur kontinuierlichen Herstellung von oligomeren, bzw. polymeren Isocyanaten durch katalytische Modifizierung monomerer Di- und/oder Triisocyanate, **dadurch gekennzeichnet, dass** mindestens eine Isocyanatkomponente A und mindestens eine Katalysatorkomponente B kontinuierlich in einem Reaktionsapparat zusammengeführt und als Reaktionsmischung durch den Reaktionsapparat geführt werden, wobei die Verweilzeitverteilung nach dem Dispersionsmodell durch Bo (Bodenstein-Zahl) oberhalb 40, bevorzugt oberhalb 60 und ganz besonders bevorzugt oberhalb 80 charakterisiert ist.

2. Verfahren gemäß Anspruch 1, **dadurch gekennzeichnet, dass** die Zusammenführung der Isocyanat- und der Katalysatorkomponente unter Verwendung eines Intensivmischers erfolgt.

3. Verfahren gemäß Anspruch 1 oder 2, **dadurch gekennzeichnet, dass** neben den Komponenten A und B weitere Verbindungen in der Reaktionsmischung enthalten sind oder dieser entlang der Reaktionsstrecke zugeführt werden, wobei es sich im ersten Fall z.B. um Haftvermittler, Stabilisatoren oder Verlaufshilfsmittel und im 2. Fall z.B. um Haftvermittler, Stabilisatoren, Verlaufshilfsmittel, von A verschiedene Di- oder Triisocyanate, von B verschiedene Katalysatoren oder um Katalysatoren deaktivierende Stoffe handelt.

4. Verfahren gemäß einem der Ansprüche 1 bis 3, **dadurch gekennzeichnet, dass** entlang der Verweilstrecke eine intensive Vermischung aller Komponenten durch Einsatz von Mischelementen erfolgt.

5. Verfahren gemäß einem der Ansprüche 1 bis 4, **dadurch gekennzeichnet, dass** es im Temperaturbereich von 0°C bis + 200°C, bevorzugt 20°C bis 180°C, besonders bevorzugt 40°C bis 120°C durchgeführt wird, wobei bevorzugt über den gesamten Umsatzbereich eine konstante Reaktionstemperatur realisiert wird.

6. Verfahren gemäß einem der Ansprüche 1 bis 5, **dadurch gekennzeichnet, dass** als Di- und/oder Triisocyanate 1,5-Pentandiisocyanat, Hexamethylendiisocyanat (HDI), 2-Methylpentan-l,5-diisocyanat, 2,4,4-Trimethyl-1,6-hexandiisocyanat, 2,2,4-Trimethyl-1,6-hexandiisocyanat, 4-Isocyanatomethyl-1,8-octandiisocyanat, 3(4)-Isocyanatomethyl-1-methylcyclohexylisocyanat (IMCI), Isophorondiisocyanat (IPDI), 1,3- sowie 1,4-Bis(isocyanatomethyl)benzen (XDI), 1,3- sowie 1,4-Bis(isocyanatomethyl)cyclohexan (H6XDI), 2,4- sowie 2,6-Toluylendiisocyanat (TDI), Bis(4-isocyanatophenyl)methan (4,4'MDI), 4-Isocyanatophenyl-2-isocyanatophenylmethan (2,4'MDI) und/oder mehrkernige Produkte, die durch Formaldehyd-Anilin-Polykondensation und anschließende Überführung der resultierenden (Poly)amine in die entsprechenden (Poly)isocyanate zugänglich sind (Polymer-MDI), eingesetzt werden.

7. Verfahren gemäß einem der Ansprüche 1 bis 5, **dadurch gekennzeichnet, dass** aliphatische Di- und/oder Triisocyanate, besonders bevorzugt aliphatische Diisocyanate, ganz besonders bevorzugt Hexamethylendiisocyanat (HDI), 2-Methylpentan-1,5-diisocyanat, 2,4,4-Trimethyl-1,6-hexandiisocyanat, 2,2,4-Trimethyl-1,6-hexandiisocyanat, 4-Isocyanatomethyl-1,8-octandiisocyanat eingesetzt werden.

8. Verfahren gemäß Verfahren gemäß einem der Ansprüche 1 bis 7, **dadurch gekennzeichnet, dass** als Katalysatoren
- Tetraorganylammonium- und/oder Tetraorganylphosphonium-salze, deren Anionen sich durch H⁺-Abstraktion aus schwachen Säuren wie z.B. Carbonsäuren, fluorierten Alkylgruppen tragenden Carbonsäuren, Phenolen, Alkoholen, Wasser und HF ableiten, in fester oder gelöster Form,
- alkoholische Lösungen von Alkali- und Erdalkali-oxiden, -hydroxiden, -alkoholaten sowie phenolaten,
- alkoholische Lösungen von Metallsalzen von Carbonsäuren, beispielsweise Kaliumacetat, Natriumbenzoat, Natriumacetat und Kaliumformiat und/oder
- tertiäre Phosphine, insbesondere solche, die das Phosphoratom als Teil eines Cyclus' enthalten, in fester oder gelöster Form,
verwendet werden.

9. Verfahren gemäß einem der Ansprüche 1 bis 8, **dadurch gekennzeichnet, dass** der Katalysator in einem Anteil zwischen 1 mol-ppm und 1 mol-%, bevorzugt zwischen 5 mol-ppm und 0,1 mol-%, bezogen auf die Masse an eingesetztem Di- und/oder Triisocyanat, eingesetzt wird.

10. Verfahren gemäß einem der Ansprüche 1 bis 9, **dadurch gekennzeichnet, dass** das Verfahren nachdem 5 bis 80 %, besonders bevorzugt 10 bis 60 %, ganz besonders bevorzugt 10 bis 45 % des eingesetzten monomeren Di-und/oder Triisocyanates umgesetzt wurden, unterbrochen werden.

11. Verfahren gemäß einem der Ansprüche 1 bis 10, **dadurch gekennzeichnet, dass** die Verweilstrecke einen maximalen Druckverlust von 0 bis 30 bar, bevorzugt 0 bis 10 bar und insbesondere bevorzugt 0 bis 4 bar und b) eine Wärmeübertragungsrate oberhalb 10 kW/(K m³), bevorzugt oberhalb 50 kW/(K m³) und besonders bevorzugt oberhalb 100 kW/(K m³) aufweist.

12. Oligomere bzw. polymere Isocyanate, erhältlich nach einem Verfahren gemäß einem der Ansprüche 1 bis 11.

13. Verwendung von oligomeren bzw. polymeren Isocyanaten gemäß Anspruch 12 zur Herstellung von ggf. geschäumten Kunststoffen, sowie Lacken, Beschichtungsmitteln, Klebstoffen und Zuschlagstoffen, bevorzugt zur Herstellung von ggf. wasserdispergierbaren Ein- und Zweikomponenten-Polyurethanlacken.

## Claims

1. Process for the continuous preparation of oligomeric or polymeric isocyanates by catalytic modification of monomeric diisocyanates and/or triisocyanates, **characterized in that** at least one isocyanate component A and at least one catalyst component B are continuously combined in a reaction apparatus and conveyed as reaction mixture through the reaction apparatus, where the residence time distribution according to the dispersion model is **characterized by** a Bo (Bodenstein number) above 40, preferably above 60 and very particularly preferably above 80.

2. Process according to Claim 1, **characterized in that** the isocyanate component and the catalyst component are combined using an intensive mixer.

3. Process according to Claim 1 or 2, **characterized in that** not only the components A and B but also further compounds are present in the reaction mixture or these are introduced along the reaction section, where these are, in the first case, for example bonding agents, stabilizers or fluidizers and in the second case, for example, bonding agents, stabilizers, fluidizers, diisocyanates or triisocyanates different from A, catalysts different from B or catalyst-deactivating materials.

4. Process according to any of Claims 1 to 3, **characterized in that** intensive mixing of all components is effected by use of mixing elements along the residence section.

5. Process according to any of Claims 1 to 4, **characterized in that** it is carried out in the temperature range from 0°C to + 200°C, preferably from 20°C to 180°C, particularly preferably from 40°C to 120°C, with a constant reaction temperature preferably being realized over the entire reaction region.

6. Process according to any of Claims 1 to 5, **characterized in that** pentane 1,5-diisocyanate, hexamethylene diisocyanate (HDI), 2-methylpentane 1,5-diisocyanate, 2,4,4-trimethylhexane 1,6-diisocyanate, 2,2,4-trimethylhexane 1,6-diisocyanate, 4-isocyanatomethyloctane 1,8-diisocyanate, 3(4)-isocyanatomethyl-1-methylcyclohexyl isocyanate (IMCI), isophorone diisocyanate (IPDI), 1,3- and 1,4-bis(isocyanatomethyl)benzene (XDI), 1,3- and 1,4-bis(isocyanatomethyl)cyclohexane (H6XDI), tolylene 2,4- and 2,6-diisocyanate (TDI), bis(4-isocyanatophenyl)methane (4,4'-MDI), 4-isocyanatophenyl-2-isocyanatophenylmethane (2,4'-MDI) and/or multiring products which can be obtained by formaldehyde-aniline polycondensation and subsequent conversion of the resulting (poly)amines into the corresponding (poly)isocyanates (polymeric MDI) are used as diisocyanates and/or triisocyanates.

7. Process according to any of Claims 1 to 5, **characterized in that** aliphatic diisocyanates and/or triisocyanates, particularly preferably aliphatic diisocyanates, very particularly preferably hexamethylene diisocyanate (HDI), 2-methylpentane 1,5-diisocyanate, 2,4,4-trimethylhexane 1,6-diisocyanate, 2,2,4-trimethylhexane 1,6-diisocyanate, 4-isocyanatomethyloctane 1,8-diisocyanate, are used.

8. Process according to processes according to any of Claims 1 to 7, **characterized in that**
- tetraorganylammonium and/or tetraorganylphosphonium salts whose anions are derived by H⁺ abstraction from weak acids such as carboxylic acids, carboxylic acids bearing fluorinated alkyl groups, phenols, alcohols, water and HF, in solid or dissolved form,
- alcoholic solutions of alkali metal and alkaline earth metal oxides, hydroxides, alkoxides and phenoxides,
- alcoholic solutions of metal salts of carboxylic acids, for example potassium acetate, sodium benzoate, sodium acetate and potassium formate and/or
- tertiary phosphines, in particular ones which contain the phosphorus atom as part of a ring, in solid or dissolved form,
are used as catalysts.

9. Process according to any of Claims 1 to 8, **characterized in that** the catalyst is used in a proportion of from 1 mol-pppm to 1 mol%, preferably from 5 mol-ppm to 0.1 mol%, based on the mass of diisocyanate and/or triisocyanate used.

10. Process according to any of Claims 1 to 9, **characterized in that** the process is stopped after from 5 to 80%, particularly preferably from 10 to 60%, very particularly preferably from 10 to 45%, of the monomeric diisocyanate and/or triisocyanate used has been reacted.

11. Process according to any of Claims 1 to 10, **characterized in that** the residence section has a maximum pressure drop of from 0 to 30 bar, preferably from 0 to 10 bar and particularly preferably from 0 to 4 bar and b) has a heat transfer rate above 10 kW/(K m³), preferably above 50 kW/(K m³) and particularly preferably above 100 kW/(K m³).

12. Oligomeric or polymeric isocyanates obtainable by a process according to any of Claims 1 to 11.

13. Use of oligomeric or polymeric isocyanates according to Claim 12 for producing optionally foamed plastics and also surface coatings, coating compositions, adhesives and auxiliaries, preferably for producing optionally water-dispersible one- and two-components polyurethane coatings.

## Revendications

1. Procédé de fabrication continue d'isocyanates oligomères ou polymères par modification catalytique de di- et/ou triisocyanates monomères, **caractérisé en ce qu'**au moins un composant isocyanate A et au moins un composant catalyseur B sont réunis en continu dans un appareil de réaction et conduits au travers de l'appareil de réaction en tant que mélange réactionnel, la distribution du temps de séjour étant **caractérisée** selon le modèle de dispersion par Bo (nombre de Bodenstein) supérieur à 40, de préférence supérieur à 60 et de manière tout particulièrement préférée supérieur à 80.

2. Procédé selon la revendication 1, **caractérisé en ce que** la réunion du composant isocyanate et du composant catalyseur a lieu en utilisant un mélangeur intensif.

3. Procédé selon la revendication 1 ou 2, **caractérisé en ce qu'**en plus des composants A et B, d'autres composés sont contenus dans le mélange réactionnel ou ceux-ci sont introduits le long de la zone de réaction, ceux-ci consistant dans le premier cas p. ex. en des promoteurs d'adhésion, des stabilisateurs ou des adjuvants d'écoulement, et dans le 2^{e} cas p. ex. en des promoteurs d'adhésion, des stabilisateurs, des adjuvants d'écoulement, des di- ou triisocyanates différents de A, des catalyseurs différents de B ou des substances désactivant les catalyseurs.

4. Procédé selon l'une quelconque des revendications 1 à 3, **caractérisé en ce qu'**un mélange intensif de tous les composants a lieu le long de la zone de séjour en utilisant des éléments de mélange.

5. Procédé selon l'une quelconque des revendications 1 à 4, **caractérisé en ce qu'**il est réalisé dans la plage de température allant de 0 °C à +200 °C, de préférence de 20 °C à 180 °C, de manière particulièrement préférée de 40 °C à 120 °C, une température de réaction constante étant de préférence réalisée sur l'ensemble de la zone de conversion.

6. Procédé selon l'une quelconque des revendications 1 à 5, **caractérisé en ce qu'**en tant que di- et/ou triisocyanates, le diisocyanate de 1,5-pentane, le diisocyanate d'hexaméthylène (HDI), le 1,5-diisocyanate de 2-méthylpentane, le diisocyanate de 2,4,4-triméthyl-1,6-hexane, le diisocyanate de 2,2,4-triméthyl-1,6-hexane, le diisocyanate de 4-isocyanatométhyl-1,8-octane, l'isocyanate de 3(4)-isocyanatométhyl-1-méthylcyclohexyle (IMCI), le diisocyanate d'isophorone (IPDI), le 1,3- et 1,4-bis(isocyanatométhyl)benzène (XDI), le 1,3- et 1,4-bis(isocyanatométhyl)cyclohexane (H6XDI), le diisocyanate de 2,4- et 2,6-toluylène (TDI), le bis(4-isocyanatophényl)méthane (4,4'MDI), le 4-isocyanatophényl-2-isocyanatophénylméthane (2,4'MDI) et/ou les produits à plusieurs noyaux qui sont accessibles par polycondensation de formaldéhyde-aniline, puis transformation des (poly)amines résultantes en les (poly)isocyanates correspondants (MDI polymère), sont utilisés.

7. Procédé selon l'une quelconque des revendications 1 à 5, **caractérisé en ce que** des di- et/ou triisocyanates aliphatiques, de manière particulièrement préférée des diisocyanates aliphatiques, de manière tout particulièrement préférée le diisocyanate d'hexaméthylène (HDI), le 1,5-diisocyanate de 2-méthylpentane, le diisocyanate de 2,4,4-triméthyl-1,6-hexane, le diisocyanate de 2,2,4-triméthyl-1,6-hexane, le diisocyanate de 4-isocyanatométhyl-1,8-octane sont utilisés.

8. Procédé selon procédés selon l'une quelconque des revendications 1 à 7, **caractérisé en ce qu'**en tant que catalyseurs,
- des sels de tétraorganylammonium et/ou de tétraorganylphosphonium, dont les anions dérivent par abstraction d'H⁺ d'acides faibles tels que p. ex. les acides carboxyliques, les acides carboxyliques portant des groupes alkyle fluorés, les phénols, les alcools, l'eau et HF, sous forme solide ou dissoute,
- des solutions alcooliques d'oxydes, d'hydroxydes, d'alcoolates et de phénolates alcalins et alcalino-terreux,
- des solutions alcooliques de sels de métaux d'acides carboxyliques, par exemple l'acétate de potassium, le benzoate de sodium, l'acétate de sodium et le formiate de potassium, et/ou
- des phosphines tertiaires, notamment celles qui contiennent l'atome de phosphore en tant que partie d'un cycle, sous forme solide ou dissoute,
sont utilisés.

9. Procédé selon l'une quelconque des revendications 1 à 8, **caractérisé en ce que** le catalyseur est utilisé en une proportion comprise entre 1 ppm en moles et 1 % en moles, de préférence entre 5 ppm en moles et 0,1 % en moles, par rapport à la masse de di- et/ou triisocyanate utilisé.

10. Procédé selon l'une quelconque des revendications 1 à 9, **caractérisé en ce que** le procédé est interrompu après la réaction de 5 à 80 %, de manière particulièrement préférée de 10 à 60 %, de manière tout particulièrement préférée de 10 à 45 %, du di- et/ou triisocyanate monomère utilisé.

11. Procédé selon l'une quelconque des revendications 1 à 10, **caractérisé en ce que** la zone de séjour présente une perte de charge maximale de 0 à 30 bar, de préférence de 0 à 10 bar et de manière particulièrement préférée de 0 à 4 bar, et b) un taux de transfert de chaleur supérieur à 10 kW/(Km³), de préférence supérieur à 50 kW/(Km³) et de manière particulièrement préférée supérieur à 100 kW/(Km³).

12. Isocyanates oligomères ou polymères, pouvant être obtenus par un procédé selon l'une quelconque des revendications 1 à 11.

13. Utilisation d'isocyanates oligomères ou polymères selon la revendication 12 pour la fabrication de plastiques éventuellement moussés, ainsi que de vernis, d'agents de revêtement, d'adhésifs et d'adjuvants, de préférence pour la fabrication de vernis de polyuréthane mono- et bicomposants éventuellement dispersibles dans l'eau.
